# EUROPEAN PATENT SPECIFICATION

(11) **EP 0 497 768 B1**
(45) Date of publication and mention of the grant of the patent: **22.10.1997**
(21) Application number: 90909231.4
(22) Date of filing: 04.06.1990
(51) Int. Cl.: A61B 19/00, A61N 5/10, G06T 15/00

(54) **PROCESS FOR USE IN RADIOSURGERY**
VERFAHREN ZUR VERWENDUNG BEI DER RADIOCHIRURGIE
PROCEDE DESTINE A LA RADIOCHIRURGIE

(43) Date of publication of application: 12.08.1992
(73) Proprietor: DOSE PLAN, INC., Glenview, IL 60025 (US)
(72) Inventor: HELENOWSKI, Tomasz, K., Glenview, IL 60025 (US)
(74) Representative: Gee, David William
(86) International application number: US9003072
(87) International publication number: WO9118644

(56) References cited:
- GB-A- 1 152 442
- US-A- 3 487 559
- US-A- 3 783 251
- US-A- 3 987 281
- US-A- 4 830 015
- JOURNAL OF DIGITAL IMAGING, vol.2, no.2, May 1989; pages 75-81; T.M. Peters et al. "Stereotactic Neurosurgery Planning on a Personal-Computer-Based Work Station"

## Description

### FIELD OF INVENTION

This invention relates to a process for aiding in the planning of radiosurgery utilising a Gamma Knife apparatus, the process including: electronically plotting a real time third dimension digitised image of the regions of interest, developing X,Y and Z coordinates from predetermined reference points on the image, and manoeuvring the image through stereoscopic space in the X,Y and Z coordinates to afford all possible lines of sight to the image.

The process includes visualizing, in real time three dimensional viewing, with the aid of a computer using steroscopic views, the treatment volume and surrounding structures. Thus, according to the invention the process includes electronically overlying the image with treatment volume imagery, and then simultaneously rotating the image and the treatment volume imagery through stereoscopic space to determine the optimum placement of the treatment volume imagery.

The treatment areas can be represented from information derived from CT scan slices or an MRI scanner both of which produce two dimensional cross sectional views of the area. These structural areas are then rotated for viewing at any angle utilizing a real time computer display. This computer enhanced display will form the steroscopic three dimensional views utilized in the radiosurgical procedure.

The treatment doses, which are units of treatment which can be produced by a Gamma knife are represented by a volume display outlining approximately the fifty percent isodose curve. These volumes, essentially spherical for the Gamma knife, are referred to as "shots", which are then through the present process moved in three dimensional space and shown in real time on three dimensional displays.

The process of the present invention is adaptable for use in stereoscopic angiography which is the showing of the blood vessels in the brain. By the employment of two simultaneously displayed stereoscopic orthogonal views, which may be rotated ninety degrees to each other, the precise locating of the X,Y, and Z coordinates can be achieved. A cursor may be moved in three dimensional space on the stereoscopic angiography display with simultaneous viewing of the cursor in both the orthogonal views. By being able to view it simultaneously in real time, projected onto both orthogonal views, precise localization of the blood vessels can be obtained.

The scan slices determined from any of the studies, CT, MRI, and angiography can be simultaneously displayed on one or more computer monitors as cross sections. Real time display of the shot approximations are shown in cross section in all of the planes simultaneously. The shots can then be moved in real time on all of these cross sections to determine how they interact within the slices and each other.

### PRIOR ART

The mechanical and electronic equipment and apparatuses employed in stereotactic surgery is presently available and their function for use in the specific field is well known in the profession.

A condensed introduction to the required physical apparatuses necessary to practice the present inventive process can be found in an article entitled **"Stereotactic Neurosurgery Planning on a PC Based Work Station"**, pages 75-81, Journal of Digital Imaging 1989, vol. 2, no. 2, on which the first part of claim 1 is based. For 3-D reconstructed CT images see **Journal Computer Assist Tomography - Vol. 12 No. 1, 1988**, Technical Note Holography of 3 D Surface Reconstructed CT Images. See also the article **Stereoscopic Computed Three-Dimensional Surface Displays; Radiographics Vol. 5 No. 6, November 1985.**

### BRIEF DESCRIPTION OF THE DRAWINGS

The inventive process described in this application will be best understood by reference to the accompanying drawings in which:
Fig. 1 is a flow chart of the step by step application of the process, and
Fig. 2 is a flow chart of the display program of the process as it is implemented.

### DESCRIPTION OF THE INVENTION

Stereotactic surgery performed through the employment of image sources which define the cerebral structure in cooperation with a computerized work station including the applicable software, is an acceptable medical procedure. The method of developing the necessary images as well as the trajectories of the shots of high energy ionizing radiation, is achieved through the use of the stereotactic frame which develops the coordinate geometry. The treatment areas are represented from information derived from modalities such as CT, MRI and DSA.

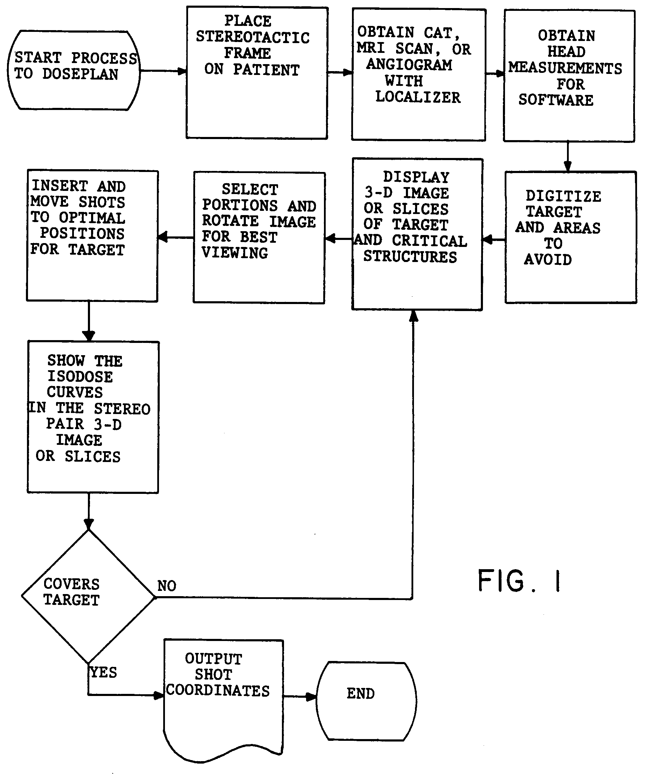

As disclosed by Fig. 1, the process begins with the placement of a stereotactic frame on the patient and through it resulting graphic points are determined. In step two these referenced graphics are overlaid with scan slices obtained through CT or MRI methods which disclose the areas to be treated in its relation to surrounding safe structure.

In step three the head measurements are then coordinated into the pre-existing software so that target areas and safe structure may be digitized. In the third step the 3-D images of target and safe structural areas are displayed. In the following step selective portions of these images may be colorized and rotated to afford all possible line of sight viewing so that prospecive shots may be optimally positioned onto the target areas, corresponding to its configuration including length, width and depth.

In the final step the perspective isodose curves are then imposed upon the images. At this stage it may be determined whether or not the shots correspond to the target's imagery. This continuous unobstructed viewing from different rotated angles allows for real time display of the target area, safe structure as well as shot positions and volumes.

The process permits the adjustment of each individual shot's volume and position enabling the operator to assure that there are no shot overlays which would produce an unwanted degree of radiation. The stereoscopic display of the shots and area to be treated affords the optimum placement of the isodose curve.

If only one graphic screen is available for display the slices around the active shot and as many additional slices as possible are displayed. If multiple graphics screens are available, all slices are displayed. Shot manipulation in real time, is displayed on the slices using the same control functions as for the third dimensional display.

The foregoing described process allows the operator to enter the outline of the treatment volume (shots) and other regions of interest (tumors or effected structure) omitting or permitting exclusion by definition critical structures that are to be spared. The display is constructed from a series of transverse axial images. These outlines are then displayed as a wire frame stereo pair image set. By utilizing a display system which allows each of the images to be viewed with the appropriate eye, the structures are displayed as a three dimensional model.

Approximations of the shots are then superimposed on this display and can be moved in stereotactic space. The entire image may then be rotated in any direction for optimal viewing of the targets and shots. Portions of the entire volume may be selected for viewing to allow better visualization of the shot positions relative to the target edges.

This novel function allows the operator to obtain a very effective approximation for the positioning of the shots to cover the target, and the ability to avoid the critical structures. After placement of the shots, their calculated isodose contours can be displayed as a stereoscopic pair superimposed on the digitized image and it to may be rotated for close examination.

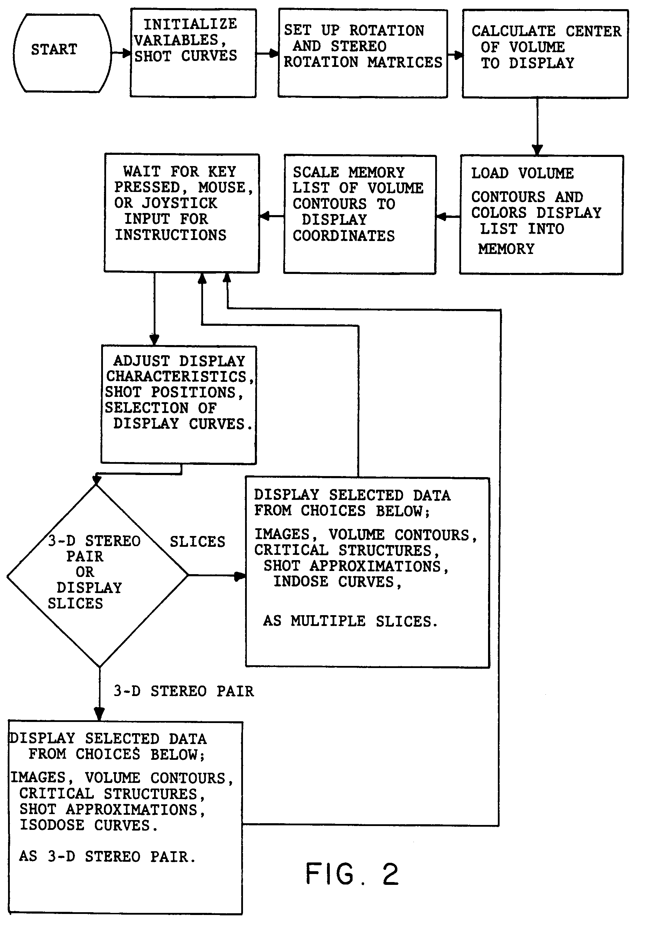

The following data, represented in the flow chart of Fig. 2 is added to the existing software and includes, as step one digitizing CT or MRI images as well as digitizing slices from the computed Axial Tomography scan/MRI digitized images, including laplace and other edge detection algorithms. Step two utilizes a mouse digitizer for selection of display curves, adjusting display characteristics as well as shot positions. This step permits the editing of curves and/or deleting curves, and will illustrate the reference distance as required.

The final step requires calculating the dose metric center from orthogonal images using prior programming, including utilizing the digitizer input from the established axes as well as the input from the dose metric center. To the following may be added a manual input of scale readings.

It is required that one must generate minimum/maximum X's, Y's for the given Z from Angio outline to correlate to the transverse axial CT or MRI images. Also to generate cross sectional plots from digitized angiographic plots to correlate to cross sections on other studies.

The dose metric center calculations may be made from AP and LAT views. Angiographic outlines are digitized from othogonal views for display. Angiographic volumes may also be digitized from othogonal views for display. Digitizing the angiographic volumes from orthogonal stereoscopic views for display through tie use of coplanar slices. Planning the angiographic volume from the orthogonal stereoscopic views using shot approximations. Generating an angio predicted outlines from CT digital slices and employing a 3-D mouse input for imagery information.

The program may include a first menu which will permit the display and adjustment of color images including color to hide imagery as well as color to display imagery; toggle display of axes; toggle hidden line removal on/off delay; color change, color coded screen labels and finally a printout instructional manual.

Another menu may include data directed to the shots such that they can be modified edited and selected for display. Each shot can be moved or all required shots may be blocked together for movement to new coordinates, a printout of shots can be obtained indicating saved shots and loaded shots with all shot information transferred to Kula.

The first menu then permits the rotation and adjustment of the images and shot volumes. It permits the display of all curves except those of the shots as well as showing the shots without the other curves so as to illustrate how they all interact. The menu will display all points outside a specific distance from the Z coordinate of the center or all shots outside specific distance from the center.

The second menu permits the activation of a cursor drone which has a variable size in the Z direction and which when activated will cause the display of the curves which fall within the Z range pointed to by the drone. The second menu will activate the graphic display between the stereoscopic 3-D display and the slices as well as simultaneously displaying the slices with shot representations imposed thereon.

While I have illustrated and described the preferred process for carrying my invention into effect, this is capable of variation and modification without departing from the invention as claimed in the appended claims.

## Claims

1. A process for aiding in the planning of radiosurgery utilising a Gamma Knife apparatus, the process including:
electronically plotting a real time third dimension digitised image of the regions of interest,
developing X,Y and Z coordinates from predetermined reference points on the said image,
manoeuvring the said image through stereoscopic space in the X,Y and Z coordinates to afford all possible lines of sight to the said image,
characterized by the steps of:
electronically overlying the said image with treatment volume imagery, and then
simultaneously rotating the said image and the said treatment volume imagery through stereoscopic space to determine the optimum placement of the treatment volume imagery.

2. A process according to claim 1 characterized by selecting portions of the regions of interest as well as sections of the treatment imagery for simultaneous movement.

3. A process according to claim 1 characterized by colouring the said displayed regions of interest as well as the treatment imagery with selected colour tones, and changing the colour tones of the selected coloured areas.

4. A process according to claim 1 characterised by selecting areas of the displayed regions of interest which have definite lines, and highlighting said definite lines of selected areas.

5. A process according to claim 1 characterized by calculating isodose contours displayed as stereoscopic pairs superimposed on the digitized image and manipulating such contours through stereoscopic space.

## Patentansprüche

1. Verfahren zur Unterstützung der Planung eines radiochirurgischen Eingriffs mit einer Gamma-Messervorrichtung, umfassend die folgenden Schritte:
elektronisches Plotten eines digitalisierten dreidimensionalen Echtzeit-Bildes des fraglichen Bereiches, Entwickeln von X-, Y- und Z-Koordinaten anhand vorbestimmter Bezugspunkte auf dem genannten Bild,
Manövrieren des genannten Bildes durch den stereoskopischen Raum in den X-, Y- und Z-Koordinaten, um alle möglichen Blicklinien auf das genannte Bild zu erhalten,
gekennzeichnet durch die folgenden Schritte:
elektronisches Überlagern des genannten Bildes mit Abbildungen des Behandlungsvolumens, und dann
gleichzeitiges Rotieren des genannten Bildes und der genannten Abbildungen des Behandlungsvolumens durch den stereoskopischen Raum, um die optimale Plazierung der Abbildungen des Behandlungsvolumens zu ermitteln.

2. Verfahren nach Anspruch 1, gekennzeichnet durch das Auswählen von Teilen der fraglichen Bereiche sowie von Abschnitten der Behandlungsabbildungen für eine gleichzeitige Bewegung.

3. Verfahren nach Anspruch 1, gekennzeichnet durch das Färben der genannten angezeigten fraglichen Bereiche sowie der Behandlungsabbildungen mit gewählten Farbtönen und Ändern der Farbtöne der gewählten gefärbten Flächen.

4. Verfahren nach Anspruch 1, gekennzeichnet durch das Auswählen von Flächen der angezeigten fraglichen Bereiche, die klare Linien aufweisen, und Hervorheben der genannten klaren Linien gewählter Flächen.

5. Verfahren nach Anspruch 1, gekennzeichnet durch das Berechnen von Isodosenkonturen, die als stereoskopische Paare angezeigt werden, die auf das digitalisierte Bild gelegt werden, und Manipulieren solcher Konturen durch den stereoskopischen Raum.

## Revendications

1. Procédé destiné à faciliter la planification de radio-chirurgie à l'aide d'un appareil à scalpel Gamma, le procédé consistant à tracer par moyen électronique une image tridimensionnelle numérisée en temps réel des régions concernées, à développer les coordonnées X, Y et Z à partir de points de référence prédéterminés sur ladite image, à manoeuvrer ladite image dans un espace stéréoscopique selon les coordonnées X, Y et Z pour obtenir tous les angles d'observation possible de ladite image, caractérisé par les opérations de : superposition par moyen électronique de ladite image sur les images de traitement de volume, puis la rotation simultané de ladite image et des images de traitement de volume dans l'espace stéréoscopique afin de déterminer la position optimale des images de traitement de volume.

2. Procédé selon la revendication 1 caractérisé par la sélection de parties des régions concernées ainsi que des coupes des images de traitement pour un mouvement simultané.

3. Procédé selon la revendication 1 caractérisé par la coloration des dites régions concernées affichées et des images de traitement avec des coloris sélectionnés, et par le changement des coloris des zones colorées sélectionnées.

4. Procédé selon la revendication 1 caractérisé par la sélection de zones des régions concernées affichées ayant des lignes précises, et par la mise en valeur des dites lignes précises des zones sélectionnées.

5. Procédé selon la revendication 1 caractérisé par le calcul des contours isodoses affichés comme paires stéréoscopiques superposées sur l'image numérisée et par la manipulation de tels contours dans l'espace stéréoscopique.
